# EUROPEAN PATENT APPLICATION

(11) **EP 4 563 091 A1**
(43) Date of publication of application: **04.06.2025**
(21) Application number: 24216600.7
(22) Date of filing: 29.11.2024
(51) Int. Cl.: A61B 8/08, A61B 8/00

(54) **ULTRASOUND DIAGNOSTIC SYSTEM**

(30) Priority: 29.11.2023 JP 2023202153
(71) Applicant: FUJI-FILM Corporation, Minato-ku Tokyo 106-8620 (JP)
(72) Inventor: SATO, Yuko, Tokyo, 106-8620 (JP)
(74) Representative: Dehns Germany Partnerschaft mbB

(57) **Abstract**

An object is to simplify setting for adjusting an image quality of an ultrasound image in an ultrasound diagnostic apparatus. The terminal apparatus (12) receives preset conditions from the user. The preset condition is a predetermined image quality parameter set related to the image quality of the ultrasound image generated by the ultrasound diagnostic apparatus (10). The terminal apparatus (12) transmits preset information indicating the preset condition to the ultrasound diagnostic apparatus (10). The ultrasound diagnostic apparatus (10) generates a plurality of pre-images from the ultrasound image in accordance with the preset condition indicated by the preset information. The ultrasound diagnostic apparatus (10) transmits the plurality of pre-images to the terminal apparatus (12). The terminal apparatus (12) displays the plurality of pre-images on the display. In a case in which the pre-image is selected from the plurality of pre-images by the user, the terminal apparatus (12) transmits information related to the selected pre-image to the ultrasound diagnostic apparatus (10).

## Description

### BACKGROUND OF THE INVENTION

### 1. Field of the Invention

The present disclosure relates to an ultrasound diagnostic system.

### 2. Description of the Related Art

JP5931416B discloses an ultrasound diagnostic apparatus that generates medical information that matches a transfer condition set by a preset content setting unit as subset data, and transfers the subset data to an external terminal connected via a communication network.

### SUMMARY OF THE INVENTION

In ultrasound diagnostic apparatus, it is not always easy for the user to perform the setting for adjusting the image quality of the ultrasound image. For example, the setting content may be changed according to a parameter for adjusting the image quality, an imaging mode, an imaging site or an imaging region, the type of the ultrasound probe, the preference of the user, and the like. Therefore, it is difficult to set the image quality adjustment to conform to the individual conditions, and it takes time and effort to make the setting.

An object of the present disclosure is to simplify setting for adjusting the image quality of an ultrasound image in an ultrasound diagnostic apparatus.

One aspect of the present disclosure relates to an ultrasound diagnostic system comprising a terminal apparatus; and an ultrasound diagnostic apparatus, in which the terminal apparatus includes a reception unit that receives a preset condition, which is a predetermined image quality parameter set related to an image quality of an ultrasound image generated by the ultrasound diagnostic apparatus, from a user, a terminal transmission unit that transmits preset information indicating the preset condition received by the reception unit to the ultrasound diagnostic apparatus, and a display control unit that displays an image on a display of the terminal apparatus, the ultrasound diagnostic apparatus includes an image generation unit that generates a plurality of pre-images from an ultrasound image in accordance with the preset condition indicated by the preset information transmitted from the terminal apparatus, and an apparatus transmission unit that transmits the plurality of pre-images to the terminal apparatus, the display control unit displays the plurality of pre-images transmitted from the ultrasound diagnostic apparatus on the display as sample images, and the terminal transmission unit transmits information related to a pre-image selected by the user from among the plurality of pre-images displayed on the display to the ultrasound diagnostic apparatus.

The display control unit may display a thumbnail image representing each of the plurality of pre-images transmitted from the ultrasound diagnostic apparatus on the display as the sample image, and the terminal transmission unit may transmit the information related to a pre-image selected by a user by selecting the thumbnail image to the ultrasound diagnostic apparatus.

The ultrasound diagnostic system further includes a storage control unit, in which the storage control unit may store at least one of history information indicating a history of an image quality adjustment by the user, subject information related to a subject on which ultrasound waves are transmitted and received in order to generate the ultrasound image for generating the pre-image, or diagnosis condition information indicating a diagnosis condition in a case in which the ultrasound image for generating the pre-image is generated, and an image quality parameter set for generating the pre-image selected by the user in association with each other in a storage device.

The terminal apparatus may further include an acquisition unit, the acquisition unit may acquire the image quality parameter set searched from the storage device by using at least one of the history information, the subject information, or the diagnosis condition information, and the display control unit may display the ultrasound image of which the image quality is adjusted in accordance with the image quality parameter set acquired by the acquisition unit on the display.

The terminal apparatus may further include a recommendation unit, the recommendation unit may specify an image quality parameter set that is recommended to the user based on the image quality parameter set for generating the pre-image selected by the user, and the history of the image quality adjustment by the user, and the display control unit may display a pre-image of which the image quality is adjusted in accordance with the image quality parameter set that is recommended to the user, on the display.

The display control unit may display information related to a change of the image quality parameter by the user on the display.

According to the present disclosure, in the ultrasound diagnostic apparatus, the setting for adjusting the image quality of the ultrasound image can be simplified.

### BRIEF DESCRIPTION OF THE DRAWINGS

Fig. 1 is a block diagram showing an example of a configuration of an ultrasound diagnostic system according to an embodiment.
Fig. 2 is a block diagram showing an example of a configuration of an ultrasound diagnostic apparatus according to an embodiment.
Fig. 3 is a block diagram showing an example of a configuration of a terminal apparatus according to the embodiment.
Fig. 4 is a diagram showing a selection screen for selecting a preset condition.
Fig. 5 is a diagram showing an example of a sample image displayed on the terminal apparatus.
Fig. 6 is a diagram showing an example of the sample image displayed on the terminal apparatus.
Fig. 7 is a diagram showing an example of the sample image displayed on the terminal apparatus.
Fig. 8 is a diagram showing an example of the sample image displayed on the terminal apparatus.
Fig. 9 is a diagram showing a pre-image.
Fig. 10 is a diagram showing the pre-image.
Fig. 11 is a diagram showing the pre-image.
Fig. 12 is a diagram showing information related to a change of an image quality parameter.
Fig. 13 is a diagram showing information related to the change of the image quality parameter.
Fig. 14 is a diagram showing information related to the change of the image quality parameter.
Fig. 15 is a diagram showing information related to the change of the image quality parameter.

### DESCRIPTION OF THE PREFERRED EMBODIMENTS

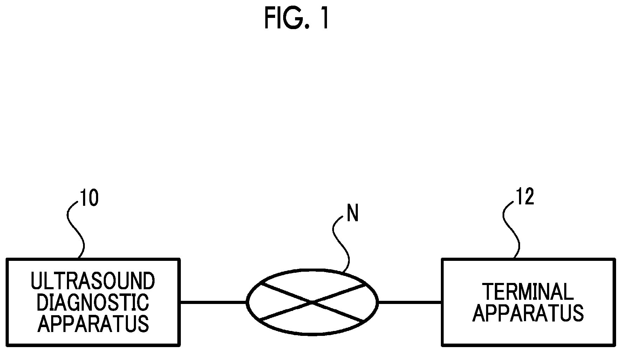

An ultrasound diagnostic system according to an embodiment will be described with reference to Fig. 1. Fig. 1 is a block diagram showing an example of a configuration of an ultrasound diagnostic system according to the embodiment.

The ultrasound diagnostic system includes one or a plurality of ultrasound diagnostic apparatuses 10 and one or a plurality of terminal apparatuses 12. The ultrasound diagnostic apparatus 10 and the terminal apparatus 12 communicate with other apparatuses via the communication path N.

The communication path N may be constructed by wireless communication or by wired communication. For example, Wi-Fi (registered trademark) or short-range wireless communication (for example, Bluetooth (registered trademark) or radio frequency identifier

(RFID)) is used as the wireless communication. The communication path N may be a network such as a local area network (LAN) or the Internet.

The ultrasound diagnostic apparatus 10 generates ultrasound image data by transmitting and receiving ultrasound waves using an ultrasound probe. For example, the ultrasound diagnostic apparatus 10 transmits the ultrasound waves into a subject and receives the ultrasound waves reflected from the inside of the subject to generate the ultrasound image data representing a tissue inside the subject.

The terminal apparatus 12 is a personal computer (hereinafter, referred to as "PC"), a tablet PC, a smartphone, a mobile phone, or the like. For example, the terminal apparatus 12 is used to set the image quality of the ultrasound image data.

For example, the image data generated by the ultrasound diagnostic apparatus 10 is transmitted to the terminal apparatus 12 via the communication path N, and the image is displayed on the terminal apparatus 12. In addition, the parameter related to the image quality input by the terminal apparatus 12 is transmitted to the ultrasound diagnostic apparatus 10 via the communication path N. The ultrasound diagnostic apparatus 10 adjusts the image quality of the ultrasound image data in accordance with the parameter transmitted from the terminal apparatus 12.

An external apparatus (for example, a server) other than the ultrasound diagnostic apparatus 10 and the terminal apparatus 12 may be included in the ultrasound diagnostic system and may communicate with the ultrasound diagnostic apparatus 10 or the terminal apparatus 12 via the communication path N.

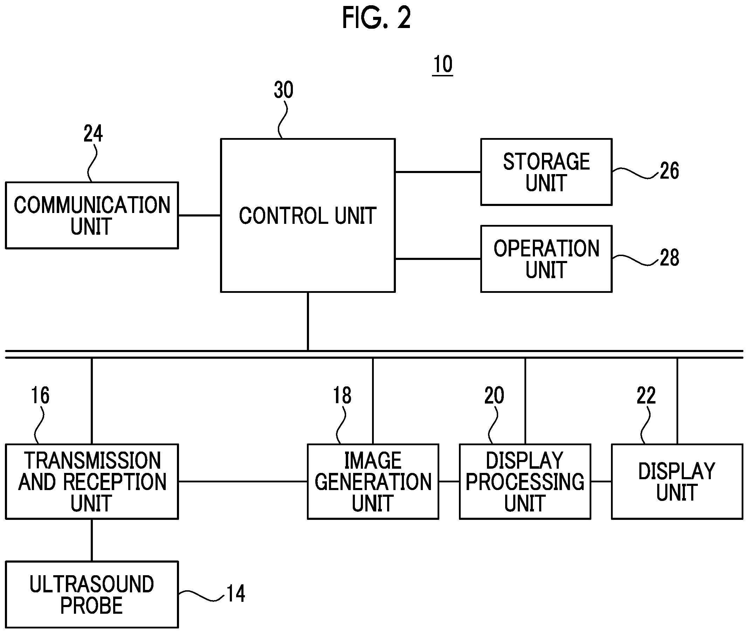

Hereinafter, the ultrasound diagnostic apparatus 10 will be described with reference to Fig. 2. Fig. 2 is a block diagram showing an example of the configuration of the ultrasound diagnostic apparatus 10.

The ultrasound probe 14 is a device that transmits and receives the ultrasound waves. The ultrasound probe 14 includes, for example, a 1D array oscillator. The 1D array oscillator includes a plurality of ultrasound oscillators arranged in one dimension. An ultrasound beam is formed by the 1D array oscillator, and electron scanning with the ultrasound beam is repeatedly performed. As a result, a scanning cross section is formed in a living body for each electron scanning. The scanning cross section corresponds to a two-dimensional echo data acquisition space. The ultrasound probe 14 may include a 2D array oscillator formed by two-dimensionally arranging a plurality of ultrasound oscillators. In a case in which the ultrasound beam is formed by the 2D array oscillator and the electron scanning with the ultrasound beam is repeatedly performed, the scanning cross section as the two-dimensional echo data acquisition space is formed for each electron scanning. In a case in which the scanning with the ultrasound beam is performed two-dimensionally, a three-dimensional space as a three-dimensional echo data acquisition space is formed. As a scanning method, sector scanning, linear scanning, convex scanning, or the like is used.

The transmission and reception unit 16 functions as a transmission beam former and a reception beam former. In the transmission, the transmission and reception unit 16 supplies a plurality of transmission signals having a certain delay relationship to the plurality of ultrasound oscillators included in the ultrasound probe 14. As a result, a transmission beam of the ultrasound waves is formed. In the reception, a reflected wave (RF signal) from the living body is received by the ultrasound probe 14, whereby a plurality of reception signals are output from the ultrasound probe 14 to the transmission and reception unit 16. The transmission and reception unit 16 forms a reception beam by applying phasing addition processing to the plurality of reception signals. The data of the reception beam is output to the image generation unit 18. That is, the transmission and reception unit 16 forms the reception beam by performing delay processing on the reception signal obtained from each ultrasound oscillator in accordance with a delay processing condition for each ultrasound oscillator and performing an addition processing on the plurality of reception signals obtained from the plurality of ultrasound oscillators. The delay processing condition is defined by reception delay data indicating a delay time. A reception delay data set (that is, a set of delay times) corresponding to the plurality of ultrasound oscillators is supplied from the control unit 30.

The electron scanning with the ultrasound beam (that is, the transmission beam and the reception beam) is performed by the action of the transmission and reception unit 16, whereby the scanning cross section is formed. The scanning cross section corresponds to a plurality of beams, and the plurality of beams constitute a reception frame (specifically, an RF signal frame). Each beam is composed of a plurality of echoes arranged in a depth direction. A plurality of reception frames arranged on a time axis are output from the transmission and reception unit 16 to the image generation unit 18 by repeating the electron scanning with the ultrasound beam. The plurality of reception frames constitute a reception frame sequence.

In a case in which the electron scanning with the ultrasound beam is performed two-dimensionally by the action of the transmission and reception unit 16, the three-dimensional echo data acquisition space is formed, and the volume data as an echo data aggregate is acquired from the three-dimensional echo data acquisition space. A plurality of volume data arranged on a time axis are output from the transmission and reception unit 16 to the image generation unit 18 by repeating the electron scanning with the ultrasound beam. The plurality of volume data constitute a volume data sequence.

The image generation unit 18 generates ultrasound image data (for example, B-mode image data) by applying signal processing such as detection, amplitude compression (for example, logarithmic compression), and conversion functions (a coordinate transformation function and an interpolation processing function by a digital scan converter (DSC)) to the reception frame output from the transmission and reception unit 16. Hereinafter, the image data will be appropriately referred to as an "image". For example, the ultrasound image data is appropriately referred to as an "ultrasound image", and the B-mode image data is appropriately referred to as a "B-mode image". It should be noted that the ultrasound image according to the present embodiment is not limited to the B-mode image, and may be any image generated by transmission and reception of ultrasound waves. For example, the ultrasound image according to the present embodiment may be a color Doppler image, a pulse Doppler image, a strain image, a shear wave elastography image, or the like.

In addition, the image generation unit 18 changes the image quality of the ultrasound image in accordance with the image quality parameter. The image quality parameter is a parameter for changing the image quality of the ultrasound image. For example, the image quality parameter is brightness, contrast, smoothening, an adaptive filter, a gamma value, sharpness, edge emphasis, gain, a frame rate, and the like. Of course, these are merely examples of the image quality parameter, and parameters other than these may be included in the category of the image quality parameter according to the present embodiment.

The image quality parameter may be preset, or may be designated by a user such as a doctor or an examination technician. For example, in a case in which the user designates the image quality parameter by operating the ultrasound diagnostic apparatus 10 or the terminal apparatus 12, the image generation unit 18 changes the image quality of the ultrasound image in accordance with the image quality parameter designated by the user.

The image quality parameter set may be determined in advance. The image quality parameter set includes a plurality of image quality parameters. For example, the image quality parameter is determined in advance for each image quality parameter such as brightness, contrast, smoothening, an adaptive filter, a gamma value, sharpness, edge emphasis, a gain, and a frame rate, and an image quality parameter set including these image quality parameters is determined.

Hereinafter, the predetermined image quality parameter set will be referred to as "preset condition". Information indicating the preset condition is referred to as "preset information". The preset information is information indicating a specific value of each image quality parameter included in the predetermined image quality parameter.

For example, the preset condition information is stored in the storage unit 26 of the ultrasound diagnostic apparatus 10, the storage unit 38 of the terminal apparatus 12, or a storage unit of the external apparatus.

For example, preset conditions are determined for each type of the ultrasound image. Specifically, preset conditions for the B-mode image, preset conditions for the color Doppler, and preset conditions for the Doppler (for example, PW or CW) are determined.

In addition, preset conditions may be determined for each type of the ultrasound probe 14, may be determined for each examination region (for example, examination site), or may be determined for each user such as a doctor or an examination technician.

For example, in a case in which the user designates the preset condition by operating the ultrasound diagnostic apparatus 10 or the terminal apparatus 12, the image generation unit 18 changes the image quality of the ultrasound image in accordance with the preset condition designated by the user. That is, the image generation unit 18 changes the image quality of the ultrasound image in accordance with each image quality parameter determined by the preset condition.

The display processing unit 20 generates a display image by overlaying necessary graphic data on the ultrasound image. The display image is output to the display unit 22. One or a plurality of images are arranged and displayed in a display aspect according to a display mode.

The display unit 22 is a display such as a liquid crystal display or an EL display. The ultrasound image such as the B-mode image is displayed on the display unit 22. The display unit 22 may be a device comprising both the display and an operation unit 28. For example, a graphic user interface (GUI) may be realized by the display unit 22 and the operation unit 28. In addition, a user interface, such as a touch panel, may be realized by the display unit 22 and the operation unit 28.

The communication unit 24 includes one or a plurality of communication interfaces including a communication chip, a communication circuit, or the like. The communication unit 24 has a function of transmitting information to another device and a function of receiving information from another device. The communication unit 24 may have a wireless communication function or a wired communication function. The communication unit 24 functions as an example of an apparatus transmission unit.

The storage unit 26 constitutes one or a plurality of storage regions for storing data. The storage unit 26 is, for example, a hard disk drive (HDD), a solid state drive (SSD), various memories (for example, RAM, DRAM, or ROM), other storage devices (for example, optical disk), or a combination thereof.

The ultrasound image generated by the transmission and reception of the ultrasound wave, information indicating the imaging condition, a parameter related to the image quality of the ultrasound image, information related to the subject (for example, the patient), and the like are stored in the storage unit 26.

The operation unit 28 is a device for the user to input a condition, a command, and the like necessary for imaging, to the ultrasound diagnostic apparatus 10. For example, the operation unit 28 is an operation panel, a switch, a button, a keyboard, a mouse, a track ball, or a joystick.

The control unit 30 controls an operation of each unit of the ultrasound diagnostic apparatus 10.

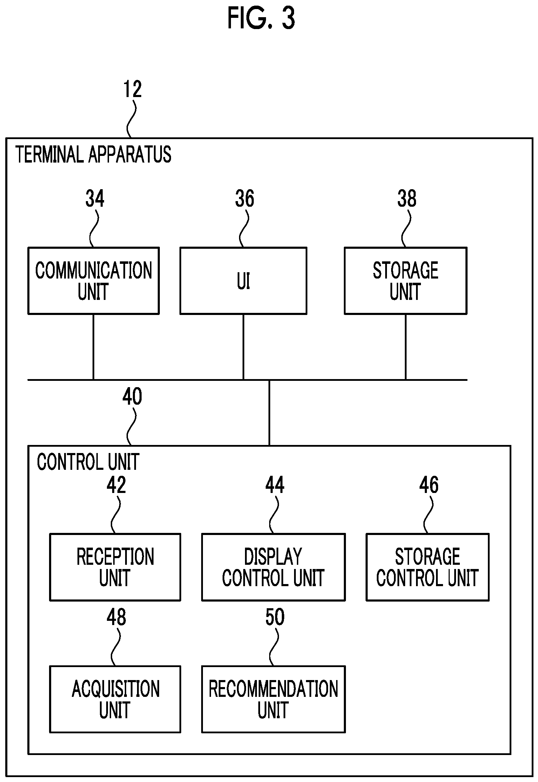

Hereinafter, the terminal apparatus 12 will be described with reference to Fig. 3. Fig. 3 is a block diagram showing an example of a configuration of the terminal apparatus 12.

The communication unit 34 includes one or a plurality of communication interfaces including a communication chip, a communication circuit, or the like. The communication unit 34 has a function of transmitting information to another device and a function of receiving information from another device. The communication unit 34 may have a wireless communication function or a wired communication function. The communication unit 34 functions as an example of a terminal transmission unit.

UI 36 is a user interface and includes a display and an operation unit. The display is a liquid crystal display, an EL display, or the like. The operation unit is a keyboard, a mouse, an input key, an operation panel, or the like. The UI 36 may be a UI such as a touch panel including both the display and the operation unit. In a case in which the UI 36 is composed of a touch panel, the information may be input through a touch pad operation. For example, the touch pad operation is a tap, a flick, a swipe, a touch and hold, a drag, a pinch-in, a pinch-out, a scrub, a pull-down, a drop, and the like.

The storage unit 38 constitutes one or a plurality of storage regions for storing data. The storage unit 38 is, for example, a hard disk drive (HDD), a solid state drive (SSD), various memories (for example, RAM, DRAM, or ROM), other storage devices (for example, optical disk), or a combination thereof.

The control unit 40 controls each unit of the terminal apparatus 12. In addition, the control unit 40 includes a reception unit 42, a display control unit 44, a storage control unit 46, an acquisition unit 48, and a recommendation unit 50.

The reception unit 42 receives the preset condition. For example, a plurality of the preset conditions are displayed on the display of the UI 36. In a case in which the user designates the preset condition by using the UI 36, the reception unit 42 receives the preset condition designated by the user.

The display control unit 44 displays various types of information on the display of the UI 36. For example, the display control unit 44 displays an image, such as an ultrasound image, on the display.

The storage control unit 46 stores various types of information in the storage unit 38 or reads out various types of information from the storage unit 38.

The acquisition unit 48 acquires the image quality parameter set from the storage device. The storage device is the storage unit 38 of the terminal apparatus 12, the storage unit 26 of the ultrasound diagnostic apparatus 10, or a storage unit of an external apparatus. In a case in which a search for the image quality parameter set is instructed in a case in which a plurality of image quality parameter sets are stored in the storage device, the acquisition unit 48 acquires the image quality parameter set that matches the search condition from the storage device.

The recommendation unit 50 specifies the image quality parameter set that is recommended to the user. The recommendation unit 50 may specify the image quality parameter set that is recommended to the user based on the history of the image quality adjustment, or may specify the image quality parameter recommended to the user by using machine learning, artificial intelligence (AI), or the like. The type of the machine learning or artificial intelligence used is not limited, and any algorithm or model may be used. For example, a convolutional neural network (CNN), a recurrent neural network (RNN), generative adversarial networks (GAN), linear models, random forests, decision tree learning, a support vector machine (SVM), an ensemble classifier, or other algorithms are used. The display control unit 44 adjusts the image quality of the ultrasound image in accordance with the image quality parameter set that is recommended to the user, and displays the ultrasound image of which the image quality is adjusted on the display of the UI 36.

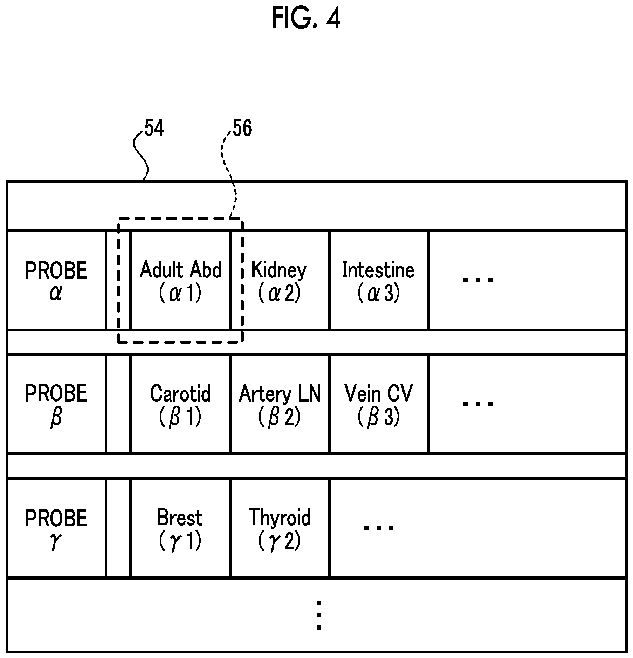

Hereinafter, the selection of the preset condition will be described with reference to Fig. 4. Fig. 4 shows a selection screen 54.

The selection screen 54 is a screen for selecting the preset condition. The selection screen 54 is displayed on the display of the UI 36 of the terminal apparatus 12. For example, in a case in which the user operates the terminal apparatus 12 to give an instruction to set the image quality parameter set, the display control unit 44 displays the selection screen 54 on the display of the UI 36. In a case in which the user operates the terminal apparatus 12 to give an instruction to display the selection screen 54, the display control unit 44 may display the selection screen 54 on the display of the UI 36.

A list of a plurality of preset conditions is displayed on the selection screen 54. Here, as an example, the preset conditions are determined for each type of the ultrasound probe 14 and for each examination region, and each preset condition is displayed on the selection screen 54.

For example, the preset conditions α1, α2, α3, ... are preset conditions suitable for the ultrasound probe α which is one type of the ultrasound probe 14. The preset condition α1 is a preset condition suitable for the examination of the abdomen. The preset condition α2 is a preset condition suitable for the examination of the kidney. The preset condition α3 is a preset condition suitable for the examination of the intestine. That is, the preset condition α1 is a preset condition suitable for the examination of the abdomen using the ultrasound probe α. The preset condition α2 is a preset condition suitable for the examination of the kidney using the ultrasound probe α. The preset condition α3 is a preset condition suitable for the examination of the intestine using the ultrasound probe α.

The same applies to the preset conditions β1, β2, β3, ..., γ1, γ2, .... That is, the preset conditions β1, β2, β3, ... are preset conditions suitable for the ultrasound probe β which is one type of the ultrasound probe 14, and are also preset conditions suitable for each of the separate examination regions. The preset conditions γ1, γ2, ... are preset conditions suitable for the ultrasound probe γ which is one type of the ultrasound probe 14, and are preset conditions suitable for each of the separate examination regions.

The preset information indicating each preset condition may be stored in advance in the storage unit 38 of the terminal apparatus 12, or may be stored in advance in the storage unit 26 of the ultrasound diagnostic apparatus 10. In a case in which the preset information is stored in the storage unit 26 of the ultrasound diagnostic apparatus 10, the terminal apparatus 12 acquires the preset information indicating each preset condition from the ultrasound diagnostic apparatus 10 via the communication path N. The display control unit 44 displays a selection screen 54 showing a list of the preset conditions on the display of the UI 36 based on preset information indicating each preset condition.

The user can select the preset condition on the selection screen 54. For example, in a case in which the selection screen 54 is displayed on the touch panel, the preset condition is selected by a touch operation or the like. In the example shown in Fig. 4, a preset condition α1 is selected by the user as indicated by reference numeral 56. The reception unit 42 receives the preset condition selected by the user.

Hereinafter, processing of setting the image quality parameter set in the ultrasound diagnostic apparatus 10 will be described.

First, in a case in which the user operates the terminal apparatus 12 to give an instruction to set the image quality parameter set, the display control unit 44 displays the selection screen 54 shown in Fig. 4 on the display of the UI 36. The preset condition in the first time may be determined for each user, or may be a preset condition determined as an initial value.

In a case in which the user selects the preset condition on the selection screen 54, the reception unit 42 receives the preset condition selected by the user. Here, as an example, the preset condition α1 is selected by the user.

The communication unit 34 of the terminal apparatus 12 transmits the preset information indicating the preset condition α1 received by the reception unit 42 to the ultrasound diagnostic apparatus 10 via the communication path N.

The communication unit 24 of the ultrasound diagnostic apparatus 10 receives the preset information transmitted from the terminal apparatus 12 via the communication path N. The image generation unit 18 of the ultrasound diagnostic apparatus 10 generates a plurality of pre-images from the basic ultrasound image in accordance with the preset condition α1 indicated by the preset information.

The basic ultrasound image may be an ultrasound image generated by actually imaging a subject (for example, a patient), or may be an ultrasound image as a standard sample image generated in advance. The standard sample image is stored in the storage unit 26 of the ultrasound diagnostic apparatus 10 or the storage unit of the external apparatus.

For example, in a case in which the subject is actually imaged by the ultrasound diagnostic apparatus 10 to generate the ultrasound image, the image generation unit 18 generates the basic ultrasound image from the reception frame in accordance with the preset condition α1. That is, the image generation unit 18 generates the basic ultrasound image according to each image quality parameter determined by the preset condition α1. The image generation unit 18 may generate the basic ultrasound image in real time, or may generate the basic ultrasound image from the reception frame stored in the storage unit 26 of the ultrasound diagnostic apparatus 10.

The pre-image is an example of a sample image used to change the image quality parameter. Specifically, the image generation unit 18 generates a plurality of ultrasound images having different image quality parameters from the basic ultrasound image. Each of the plurality of ultrasound images corresponds to a pre-image. For example, the image generation unit 18 generates a plurality of pre-images having different brightnesses by changing the brightness of the basic ultrasound image. In addition, the image generation unit 18 generates a plurality of pre-images having different contrasts by changing the contrast of the basic ultrasound image. In addition, the image generation unit 18 generates a plurality of pre-images having different sharpnesses by changing the sharpness of the basic ultrasound image. In addition, the image generation unit 18 generates a plurality of pre-images having different gamma values by changing the gamma value of the basic ultrasound image. The same applies to the other image quality parameters.

The image generation unit 18 associates pre-image identification information for identifying a pre-image with the pre-image for each pre-image. In addition, the image generation unit 18 associates information indicating the image quality parameter used to generate the pre-image with the pre-image for each pre-image. For example, in the pre-image in which the value of the contrast is "C0" and the value of the brightness is "B0", the value of the contrast "C0" and the value of the brightness "B0" are associated with each other. The same applies to the other pre-images and the other image quality parameters. For example, the control unit 30 stores the pre-image in which the pre-image identification information and the information indicating the image quality parameter are associated with each other in the storage unit 26. As a result, the pre-image identification information and the information indicating the image quality parameter are associated with each other.

The communication unit 24 of the ultrasound diagnostic apparatus 10 transmits the plurality of pre-images to the terminal apparatus 12 via the communication path N. Each pre-image is transmitted from the ultrasound diagnostic apparatus 10 to the terminal apparatus 12 in a state in which the pre-image identification information and the information indicating the image quality parameter are associated with each other.

The communication unit 34 of the terminal apparatus 12 receives the plurality of pre-images transmitted from the ultrasound diagnostic apparatus 10. The display control unit 44 displays the plurality of pre-images on the display of the UI 36 as the sample image.

The user can select the image quality parameter used to generate the selected pre-image by selecting the pre-image from the plurality of pre-images displayed.

In a case in which the user selects the pre-image from among the plurality of pre-images displayed by operating the terminal apparatus 12, the communication unit 34 of the terminal apparatus 12 transmits information related to the pre-image selected by the user to the ultrasound diagnostic apparatus 10 via the communication path N.

The information related to a pre-image may be information indicating the image quality parameter used to generate the pre-image (that is, information indicating the image quality parameter associated with the pre-image), may be pre-image identification information of the pre-image (that is, pre-image identification information associated with the pre-image), or may be both the information indicating the image quality parameter and the pre-image identification information.

The communication unit 24 of the ultrasound diagnostic apparatus 10 receives the information related to the pre-image transmitted from the terminal apparatus 12 via the communication path N.

The control unit 30 of the ultrasound diagnostic apparatus 10 sets the image quality parameter set as a new preset condition in the ultrasound diagnostic apparatus 10 based on the information related to the pre-image transmitted from the terminal apparatus 12.

In a case in which the information related to the pre-image is information indicating the image quality parameter, the control unit 30 generates the new preset condition by changing the image quality parameter included in the preset condition used to generate the basic ultrasound image to the image quality parameter indicated by the information related to the pre-image. In the example described above, since the preset condition α1 is selected by the user, the control unit 30 generates the new preset condition from the preset condition α1 by changing the image quality parameter included in the preset condition α1 to the image quality parameter indicated by the information related to the pre-image. The new preset condition is set in the ultrasound diagnostic apparatus 10.

In a case in which the information related to the pre-image is the pre-image identification information, the control unit 30 generates new preset conditions by changing the image quality parameter included in the preset conditions used to generate the basic ultrasound image to the image quality parameter associated with the pre-image identification information. The new preset condition is set in the ultrasound diagnostic apparatus 10.

Hereinafter, the image generation unit 18 of the ultrasound diagnostic apparatus 10 generates the ultrasound image in accordance with the set new preset condition. That is, the image generation unit 18 adjusts the image quality of the ultrasound image in accordance with each image quality parameter determined by the new preset condition.

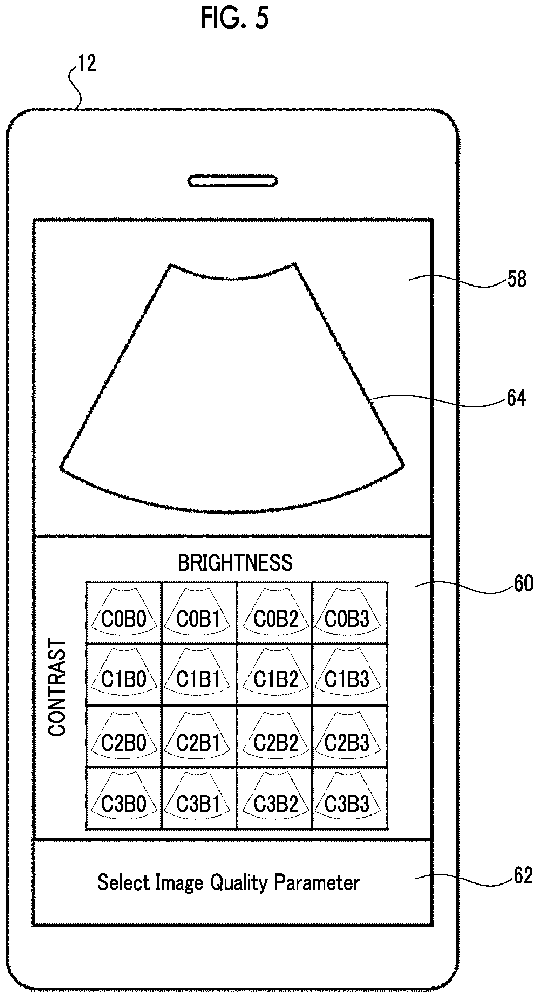

Hereinafter, a display example of a sample image will be described with reference to Figs. 5 to 8. Figs. 5 to 8 show an example of a sample image displayed on the terminal apparatus 12. Here, as an example, the terminal apparatus 12 is a tablet PC or a smartphone. In addition, it is assumed that the preset condition α1 is selected by the user.

As shown in Figs. 5 to 8, the display control unit 44 of the terminal apparatus 12 forms display regions 58, 60, and 62 on the display of the UI 36. The display regions 58, 60, and 62 are the screens on the display of the UI 36.

The display region 58 is a region in which the ultrasound image 64 is displayed. For example, the ultrasound image 64 is transmitted from the ultrasound diagnostic apparatus 10 to the terminal apparatus 12, and the display control unit 44 displays the ultrasound image 64 in the display region 58. The ultrasound image 64 may be an ultrasound image generated in real time by the ultrasound diagnostic apparatus 10, or may be a standard sample image generated in advance. For example, the ultrasound image 64 is generated in accordance with the preset condition α1 and displayed in the display region 60.

The display region 60 is a region in which a plurality of pre-images as sample images are displayed. For example, the display control unit 44 displays a thumbnail image representing each of the plurality of pre-images in the display region 60.

The display region 62 is a region in which a message, a button, or the like is displayed. For example, a button or the like for selecting the pre-image is displayed in the display region 62.

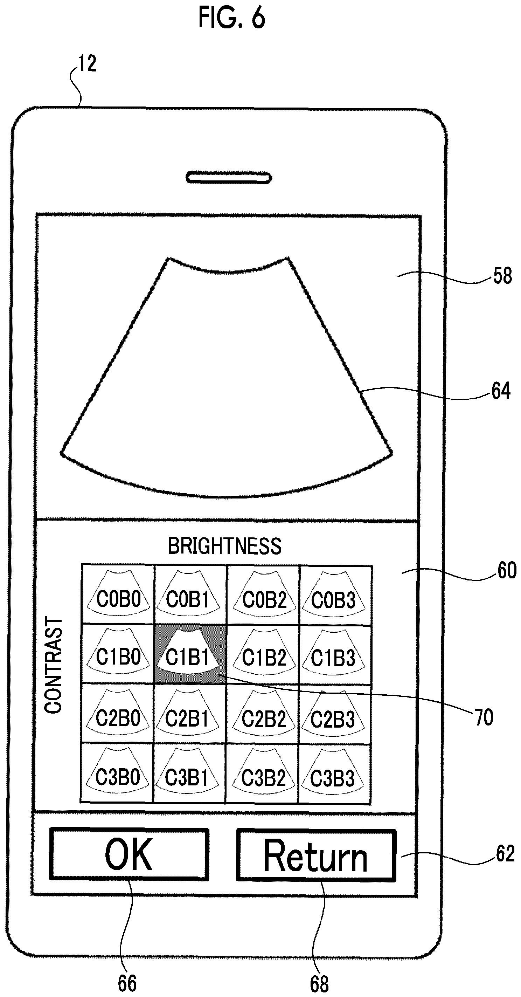

In the examples shown in Figs. 5 and 6, a plurality of pre-images having different contrasts and brightnesses are generated, and each pre-image is displayed in the display region 60 as a thumbnail image. Specifically, each of the pre-images C0B0 to C3B3 is displayed in the display region 60 as the thumbnail image. Each of the symbols "C0" to "C3" indicates the contrast, and each of the symbols "B0" to "B3" indicates the brightness. For example, the pre-image C0B0 is a pre-image having a contrast of "C0" and a brightness of "B0". The same applies to the other pre-images.

For example, the image generation unit 18 generates each of the pre-images C0B0 to C3B3 by changing the contrast of the basic ultrasound image to any of the contrasts "C0" to "C3" and changing the brightness of the basic ultrasound image to any of the brightnesses "B0" to "B3". That is, the image generation unit 18 generates each of the pre-images C0B0 to C3B3 by changing the contrast included in the preset condition α1 to any of the contrasts "C0" to "C3" and changing the brightness included in the preset condition α1 to any of the brightnesses "B0" to "B3"

For example, the image generation unit 18 generates the pre-image C0B0 by changing the contrast of the basic ultrasound image to the contrast "C0" and changing the brightness of the basic ultrasound image to the brightness "B0". That is, the image generation unit 18 generates the pre-image C0B0 by changing the contrast included in the preset condition α1 to the contrast "C0" and changing the brightness included in the preset condition α1 to the brightness "B0". The same applies to the other pre-images.

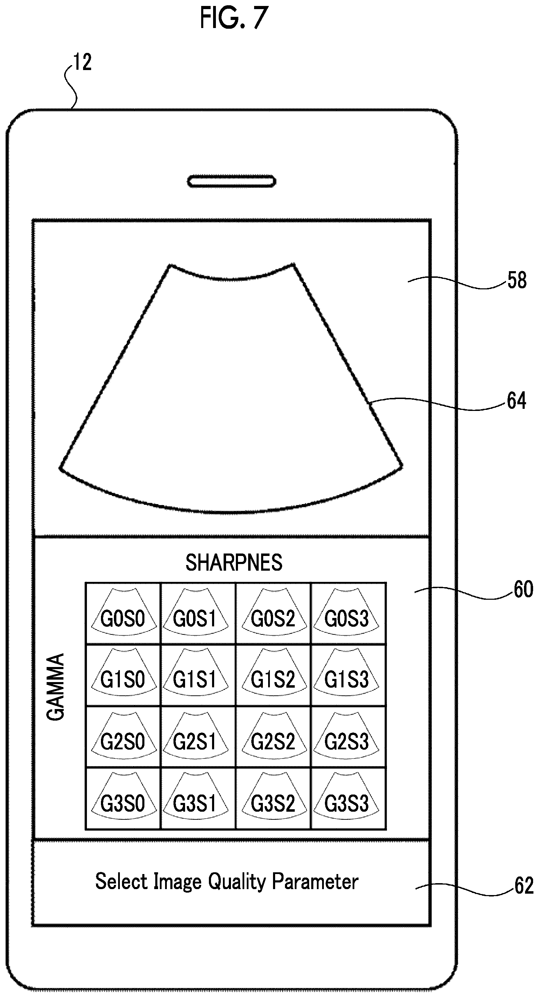

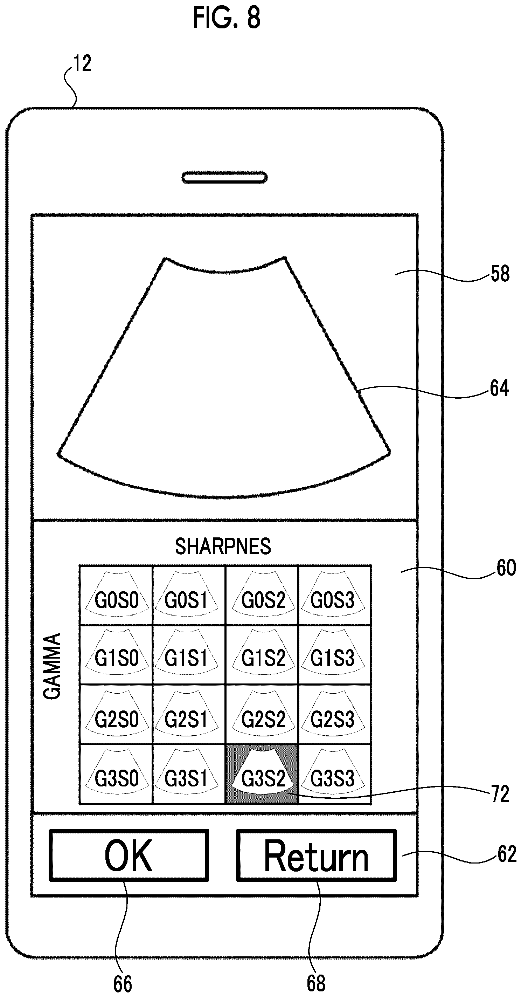

In the examples shown in Figs. 7 and 8, a plurality of pre-images having different gamma values and sharpnesses are generated, and each pre-image is displayed in the display region 60 as a thumbnail image. Specifically, each of the pre-images G0S0 to G3S3 is displayed in the display region 60 as the thumbnail image. Each of the symbols "G0" to "G3" indicates a gamma value, and each of the symbols "S0" to "S3" indicates a sharpness. For example, the pre-image G0S0 is a pre-image in which the gamma value is the gamma value "G0" and the sharpness is the sharpness "S0". The same applies to the other pre-images.

For example, the image generation unit 18 generates each of the pre-images G0S0 to G3 S3 by changing the gamma value of the basic ultrasound image to any of the gamma values "G0" to "G3" and changing the sharpness of the basic ultrasound image to any of the sharpnesses "S0" to "S3". That is, the image generation unit 18 generates each of the pre-images G0S0 to G3S3 by changing the gamma value included in the preset condition α1 to any of the gamma values "G0" to "G3" and changing the sharpness included in the preset condition α1 to any of the sharpnesses "S0" to "S3".

For example, the image generation unit 18 generates the pre-image G0S0 by changing the gamma value of the basic ultrasound image to a gamma value "G0" and changing the sharpness of the basic ultrasound image to a sharpness "S0". That is, the image generation unit 18 generates the pre-image G0S0 by changing the gamma value included in the preset condition α1 to the gamma value "G0" and changing the sharpness included in the preset condition α1 to the sharpness "S0". The same applies to the other pre-images.

For example, each pre-image is an image generated by using a plurality of image quality parameters in a trade-off relationship with each other. Specifically, as an example, each image quality parameter such as contrast, brightness, a gamma value, and sharpness is in a trade-off relationship with each other.

The user can select the image quality parameter used to generate a pre-image (that is, the image quality parameter associated with the pre-image) by selecting the pre-image on the display region 60.

In the example shown in Fig. 6, as indicated by a reference numeral 70, the user selects the pre-image C1B1. As a result, the contrast "C1" and the brightness "B1" are selected by the user. In a case in which the user selects the pre-image C1B1, the display control unit 44 may adjust the contrast of the ultrasound image 64 to the contrast "CF" and adjust the brightness of the ultrasound image 64 to the brightness "B 1". In this case, the display control unit 44 displays the ultrasound image 64 in which the contrast and the brightness are adjusted in the display region 58.

In a case in which the pre-image is selected by the user, the display control unit 44 displays the OK button 66 and the Return button 68 in the display region 62. In a case in which the OK button 66 is selected by the user, the communication unit 34 of the terminal apparatus 12 transmits the information related to the pre-image selected by the user to the ultrasound diagnostic apparatus 10 via the communication path N. In a case in which the Return button 68 is selected by the user, the selection of the pre-image is canceled.

In a case in which the user selects the pre-image C1B1 and presses the OK button 66, the communication unit 34 of the terminal apparatus 12 transmits the information related to the pre-image C1B1 to the ultrasound diagnostic apparatus 10 via the communication path N. For example, the communication unit 34 transmits information indicating the contrast "C1" and the brightness "B1" to the ultrasound diagnostic apparatus 10 as information related to the pre-image C1B1. As another example, the communication unit 34 may transmit the pre-image identification information of the pre-image C1B1 to the ultrasound diagnostic apparatus 10 as the information related to the pre-image C1B1.

The control unit 30 of the ultrasound diagnostic apparatus 10 generates new preset conditions from the preset conditions α1 by changing the contrast included in the preset conditions α1 to the contrast "C1" and changing the brightness included in the preset conditions α1 to the brightness "B1". The control unit 30 sets the new preset condition in the ultrasound diagnostic apparatus 10. Hereinafter, the ultrasound diagnostic apparatus 10 generates the ultrasound image according to the set new preset condition.

In the example shown in Fig. 8, the user selects the pre-image G3S2 as indicated by the reference numeral 72. As a result, the gamma value "G3" and the sharpness "S2" are selected by the user. In a case in which the user selects the pre-image G3S2, the display control unit 44 may adjust the gamma value of the ultrasound image 64 to the gamma value "G3" and may adjust the sharpness of the ultrasound image 64 to the sharpness "S2". In this case, the display control unit 44 displays the ultrasound image 64 in which the gamma value and the sharpness are adjusted in the display region 58.

In a case in which the user selects the pre-image G3S2 and presses the OK button 66, the communication unit 34 of the terminal apparatus 12 transmits the information related to the pre-image G3S2 to the ultrasound diagnostic apparatus 10 via the communication path N. For example, the communication unit 34 transmits the information indicating the gamma value "G3" and the sharpness "S2" to the ultrasound diagnostic apparatus 10 as the information related to the pre-image G3S2. As another example, the communication unit 34 may transmit the pre-image identification information of the pre-image G3 S2 to the ultrasound diagnostic apparatus 10 as the information related to the pre-image G3S2.

The control unit 30 of the ultrasound diagnostic apparatus 10 generates a new preset condition from the preset condition α1 by changing the gamma value included in the preset condition α1 to the gamma value "G3" and changing the sharpness included in the preset condition α1 to the sharpness "S2". The control unit 30 sets the new preset condition in the ultrasound diagnostic apparatus 10. Hereinafter, the ultrasound diagnostic apparatus 10 generates the ultrasound image according to the set new preset condition.

For example, as shown in Fig. 6, in a case in which the pre-image C1B1 is selected and the OK button 66 is pressed, the display control unit 44 displays the pre-images G0S0 to G3S3 in the display region 60, as shown in Fig. 7.

As shown in Figs. 6 and 8, in a case where the contrast "C1", the brightness "B1", the gamma value "G3", and the sharpness "S2" are selected by the user, the control unit 30 of the ultrasound diagnostic apparatus 10 generates a new preset condition α1' by changing the image quality parameters included in the preset condition α1 to the image quality parameters selected by the user. Hereinafter, the ultrasound diagnostic apparatus 10 generates the ultrasound image according to the new preset condition α1'.

For example, the ultrasound image generated in accordance with the new preset condition α1' is transmitted from the ultrasound diagnostic apparatus 10 to the terminal apparatus 12 and displayed on the display of the UI 36 of the terminal apparatus 12. For example, the ultrasound image is displayed in the display region 58. The user can confirm whether or not the ultrasound image having the image quality intended by the user is generated by referring to the ultrasound image. In a case in which the ultrasound image having the image quality intended by the user is generated, the ultrasound examination is performed by using the preset condition α1'. In a case in which the ultrasound image having the image quality intended by the user is not generated, the user re-selects or repeats the selection of the pre-image. For example, the pre-image is selected repeatedly until the ultrasound image having the image quality intended by the user is generated.

The selection of the pre-image, the pressing of the OK button 66, and the pressing of the Return button 68 may be performed by voice instructions.

As described above, by displaying the plurality of pre-images having different image quality parameters as the sample images on the terminal apparatus 12, the user of the terminal apparatus 12 can visually confirm and select the image quality parameter. As a result, the setting for adjusting the image quality of the ultrasound image can be simplified.

As an application scene of the ultrasound diagnostic system according to the present embodiment, the following application scenes can be considered.

For example, a doctor executes the ultrasound examination by applying the ultrasound probe 14 to the patient. In this case, the technician of the ultrasound diagnostic apparatus 10 (for example, a technician who performs maintenance or the like) operates the terminal apparatus 12 to adjust the image quality parameter set set in the ultrasound diagnostic apparatus 10 such that the ultrasound image having the image quality intended by the doctor is generated. The technician selects the preset condition and the pre-image by operating the terminal apparatus 12. For example, the preset condition is selected or the pre-image is selected until the ultrasound image having the image quality intended by the doctor is generated. According to the present embodiment, the technician can adjust the image quality parameter set in the ultrasound diagnostic apparatus 10 without directly operating the ultrasound diagnostic apparatus 10. For example, the technician can adjust the image quality parameter without hindering the operation of the doctor who directly operates the ultrasound diagnostic apparatus 10.

Since the image quality parameter can be adjusted by using the terminal apparatus 12, the image quality parameter may be adjusted by using the terminal apparatus 12 in a place adjacent to the ultrasound diagnostic apparatus 10, or may be adjusted in a place different from a place where the ultrasound diagnostic apparatus 10 is installed. For example, even in a case in which the ultrasound examination is actually performed on the patient, the image quality parameter can be adjusted using the terminal apparatus 12 in another room or the like. As a result, the privacy of the patient is protected.

The image quality parameter set for each of the plurality of ultrasound diagnostic apparatuses 10 may be adjusted by one terminal apparatus 12. Of course, the image quality parameter set in one ultrasound diagnostic apparatus 10 may be adjusted by one terminal apparatus 12.

Hereinafter, the processing by the storage control unit 46 and the acquisition unit 48 will be described.

The storage control unit 46 stores at least one of the history information, the subject information, or the diagnosis condition information, and the image quality parameter set for generating the pre-image selected by the user in association with each other in a storage device (hereinafter, referred to as a "history storage device" for convenience).

The history information is information indicating a history of the image quality adjustment by the user. The history of the image quality adjustment is a history of the change of the image quality parameter by the user. For example, the history of image quality adjustment is a history indicating which image quality parameter is changed to what value by the user at what time. For example, the storage control unit 46 associates and includes, in the history information, for each image quality parameter, the value of the image quality parameter before the change (that is, the image quality parameter before the change), the value of the image quality parameter after the change (that is, the image quality parameter after the change), information for identifying the user who has changed the image quality parameter, and information indicating the date and time at which the image quality parameter is changed.

The history of the image quality adjustment may be a history of a change in the image quality parameter set by the user. For example, the history of image quality adjustment is a history indicating which image quality parameter set is changed to what value by the user at what time. For example, the storage control unit 46 associates and includes, in the history information, for each image quality parameter set, the value of the image quality parameter set before the change (that is, the image quality parameter set before the change), the value of the image quality parameter set after the change (that is, the image quality parameter set after the change), the information for identifying the user who has changed the image quality parameter set, and the information indicating the date and time at which the image quality parameter set is changed.

The subject information is information related to the subject on which the ultrasound waves are transmitted and received in order to generate the ultrasound image for generating the pre-image (for example, the basic ultrasound image). For example, the subject information includes subject identification information (for example, a patient ID or the like) for identifying the subject. The subject information may include information indicating an age and a sex of the subject. For example, in a case in which the ultrasound examination is performed using the ultrasound diagnostic apparatus 10, the subject information is input to the ultrasound diagnostic apparatus 10 via the operation unit 28 or the communication path N. The input subject information is transmitted from the ultrasound diagnostic apparatus 10 to the terminal apparatus 12 via the communication path N, and the storage control unit 46 receives the subject information.

The diagnosis condition information includes information indicating a site at which the ultrasound examination is performed, information indicating an imaging mode of the ultrasound wave, information indicating a type of the ultrasound probe 14, and the like.

The image quality parameter set for generating the pre-image selected by the user is a preset condition generated by changing the preset condition selected by the user according to the image quality parameter associated with the pre-image selected by the user. In a case in which the preset condition α1, the pre-image C1B1, and the pre-image G3S2 are selected by the user, the preset condition α1' is the image quality parameter set for generating the pre-image selected by the user. That is, the preset condition α1' generated by changing the combination of the contrast and the brightness included in the preset condition α1 to the combination of the contrast "C1" and the brightness "B1" and changing the combination of the gamma value and the sharpness to the combination of the gamma value "G3" and the sharpness "S2" is the image quality parameter set for generating the pre-image selected by the user.

The history storage device may be the storage unit 38 of the terminal apparatus 12, the storage unit 26 of the ultrasound diagnostic apparatus 10, or a storage unit included in an external apparatus, such as a server. At various facilities such as hospitals, at least one of the history information, the subject information, or the diagnosis condition information, and the image quality parameter set for generating the pre-image selected by the user may be associated with each other, and may be stored in a history storage device provided in an external apparatus such as a server.

For example, at least one of the history information, the subject information, or the diagnosis condition information is determined in advance, and the storage control unit 46 stores the predetermined information and the image quality parameter set used to generate the pre-image selected by the user in association with each other in the history storage device. The user may select the information stored in the history storage device from the history information, the subject information, and the diagnosis condition information.

In addition, the storage control unit 46 may store at least one of the history information, the subject information, or the diagnosis condition information with the image quality parameter set used to generate the pre-image selected by the user, and the audio information in association with each other in the history storage device. For example, the voice is collected by the microphone provided in the terminal apparatus 12 to generate the voice information. For example, in a case in which the user issues a voice in a case in which the pre-image is selected, the microphone collects the voice of the user, and the storage control unit 46 stores the voice information indicating the voice in the history storage device in association with the other information. In this manner, it is possible to understand the situation in a case in which the pre-image is selected later by confirming the voice.

The acquisition unit 48 acquires the image quality parameter set searched from the history storage device by using at least one of the history information, the subject information, or the diagnosis condition information. The display control unit 44 displays the ultrasound image of which the image quality is adjusted in accordance with the image quality parameter set acquired by the acquisition unit 48 on the display of the UI 36.

For example, the user operates the terminal apparatus 12 to input at least one of the history information, the subject information, or the diagnosis condition information as the search key. The acquisition unit 48 searches for and acquires the image quality parameter set associated with the input search key from among the information stored in the history storage device.

The display control unit 44 adjusts the image quality of the ultrasound image in accordance with the image quality parameter set acquired by the acquisition unit 48, and and displays the ultrasound image of which the image quality is adjusted on the display of the UI 36. For example, the ultrasound image is generated in real time by the ultrasound diagnostic apparatus 10 and transmitted to the terminal apparatus 12. The display control unit 44 adjusts the image quality of the ultrasound image transmitted from the ultrasound diagnostic apparatus 10 in accordance with the image quality parameter set acquired by the acquisition unit 48. Of course, the display control unit 44 may acquire the ultrasound image stored in the storage unit 26 of the ultrasound diagnostic apparatus 10, the storage unit 38 of the terminal apparatus 12, or the external apparatus, and may adjust the image quality of the acquired ultrasound image in accordance with the image quality parameter set acquired by the acquisition unit 48. The display control unit 44 displays the ultrasound image of which the image quality is adjusted on the display of the UI 36.

For example, in a case where preset conditions used in another facility are stored in the history storage device, the user can search for the preset conditions used in the other facility from the history storage device and can adjust the image quality of the ultrasound image by using the preset conditions. In this way, the user can adjust the image quality of the ultrasound image with reference to the preset condition used in another facility. That is, the image quality parameter set can be shared by the plurality of users.

In the example shown in Fig. 3, the terminal apparatus 12 includes the storage control unit 46 and the acquisition unit 48, but the ultrasound diagnostic apparatus 10 may include the storage control unit 46 and the acquisition unit 48. In a case where the ultrasound diagnostic apparatus 10 includes the storage control unit 46 and the acquisition unit 48, the terminal apparatus 12 may not include the storage control unit 46 and the acquisition unit 48. For example, the acquisition unit 48 of the ultrasound diagnostic apparatus 10 may search the image quality parameter set from the history storage device, and the image generation unit 18 may adjust the image quality of the ultrasound image in accordance with the searched image quality parameter set. The ultrasound image of which the image quality is adjusted is displayed on the display unit 22.

Hereinafter, the processing by the recommendation unit 50 will be described.

The recommendation unit 50 specifies the image quality parameter set that is recommended to the user based on the image quality parameter set for generating the pre-image selected by the user, and the history of the image quality adjustment by the user.

As described above, the history information indicating the history of the image quality adjustment by the user is stored in the history storage device. For example, as shown in Figs. 6 and 8, in a case in which the pre-image is selected by the user, the recommendation unit 50 specifies the image quality parameter set that is recommended to the user based on the image quality parameter set for generating the selected pre-image, and the history of the image quality adjustment indicated by the history information stored in the history storage device. For example, in a case in which there is a history that the image quality parameter set is changed from the image quality parameter set A to the image quality parameter set B in a case in which the image quality parameter set for generating the pre-image selected by the user is the image quality parameter set A, the recommendation unit 50 specifies the image quality parameter set B as the image quality parameter set that is recommended to the user.

As another example, the learning device may be generated by learning the selection history of the image quality parameter set, the selection history of the pre-image, the history of the image quality adjustment, the diagnosis conditions, and the like by machine learning or the like. The recommendation unit 50 inputs the image quality parameter set for generating the pre-image selected by the user to the learning device, and acquires the image quality parameter set as the output corresponding to the input from the learning device as the image quality parameter that is recommended to the user. The data of the learning device may be stored in the storage unit 38 of the terminal apparatus 12, may be stored in the storage unit 26 of the ultrasound diagnostic apparatus 10, or may be stored in a storage unit of an external apparatus such as a server.

The display control unit 44 adjusts the image quality of the ultrasound image in accordance with the image quality parameter set specified by the recommendation unit 50. The display control unit 44 displays the ultrasound image of which the image quality is adjusted on the display of the UI 36.

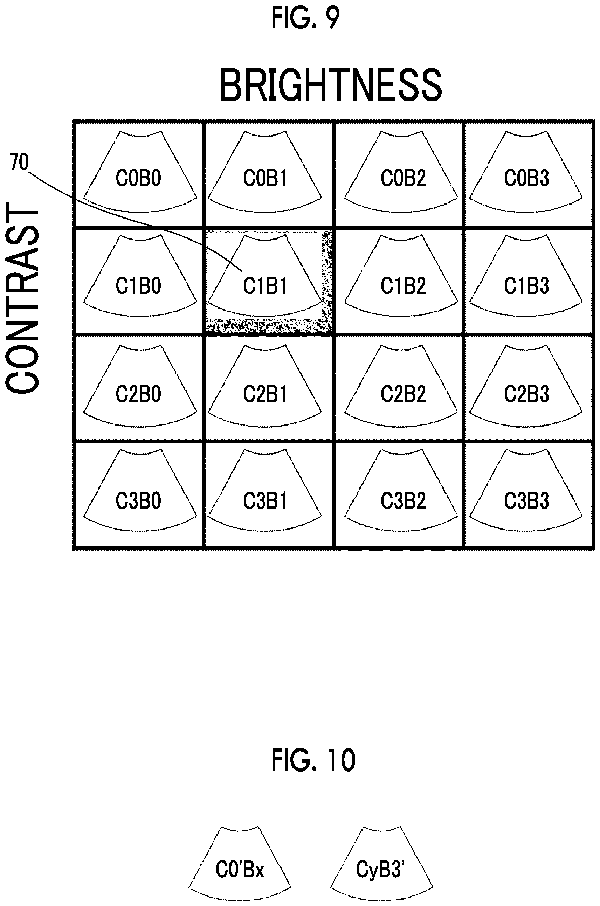

Hereinafter, a specific example of the processing by the recommendation unit 50 will be described with reference to Figs. 9 to 11. Figs. 9 to 11 show the pre-images.

Fig. 9 shows the pre-images C0B0 to C3B3. Although the display region 60 is not shown in Fig. 9, the pre-images C0B0 to C3B3 are displayed in the display region 60 of the terminal apparatus 12 as in the example shown in Fig. 6. Here, as an example, the pre-image C1B1 is selected by the user.

For example, the recommendation unit 50 specifies the image quality parameter set that is recommended to the user based on the image quality parameter set for generating the pre-image C1B1 (image quality parameter set including the contrast "C1" and the brightness "B1"), and the history of the image quality adjustment indicated by the history information stored in the history storage device.

As another example, the recommendation unit 50 may input the image quality parameter set for generating the pre-image C1B1 to the learning device, and may acquire the image quality parameter set as the output corresponding to the input from the learning device as the image quality parameter that is recommended to the user.

The pre-image C0'Bx and the pre-image CyB3' are shown in Fig. 10. The pre-image CO'Bx and the pre-image CyB3' are pre-images that is recommended to the user (that is, sample images). The pre-image CO'Bx and the pre-image CyB3' are pre-images generated in accordance with the image quality parameter set that is recommended to the user. That is, the contrast "C0'" and the brightness "B3'" are the image quality parameters that is recommended to the user.

The user who has selected the image quality parameter set including the contrast "C1" and the brightness "B1" in the past tends to change the contrast to the contrast "C0'" and to change the brightness to "B3"'. For example, a learning device is generated by training the learning device with the history of the change. The recommendation unit 50 specifies the image quality parameter set that is recommended to the user by using the learning device.

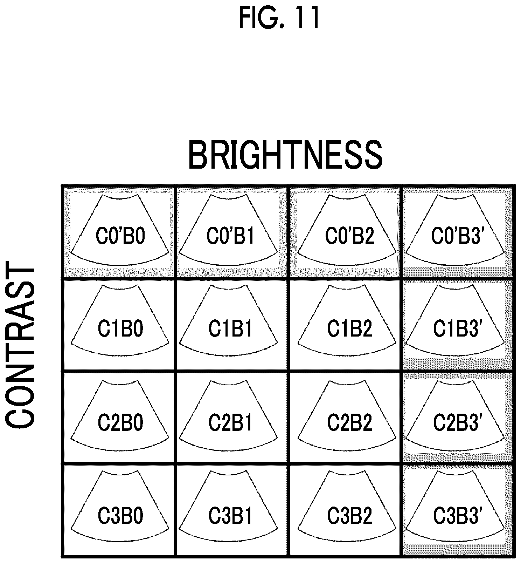

The display control unit 44 displays a plurality of pre-images including the pre-image CO'Bx and the pre-image CyB3' in the display region 60. Fig. 11 shows a display example thereof. Although the display region 60 is not shown in Fig. 11, the display control unit 44 displays the plurality of pre-images shown in Fig. 11 in the display region 60 in the same manner as in the example shown in Fig. 6.

In the example shown in Fig. 11, the display control unit 44 displays the pre-images C0'B0 to C0'B2 and C0'B3' to C3B3' in the display region 60 instead of the pre-images C0B0 to C0B3 and C1B3 to C3B3 (see Fig. 6). The other pre-images are the same as the pre-images shown in Fig. 6.

From the next time, the user selects the pre-image having the contrast and the brightness intended by the user from among the plurality of pre-images shown in Fig. 11. As a result, the user can select the pre-image having the image quality intended by the user from among the plurality of pre-images that are recommended to the user. That is, the user can select the image quality parameter set that can generate the image quality intended by the user from among the plurality of image quality parameter sets that are recommended to the user.

The display control unit 44 may display information related to the change of the image quality parameter by the user on the display of the UI 36. For example, the display control unit 44 displays the image quality parameter before the change and the image quality parameter after the change on the display as information related to the change of the image quality parameter. As another example, the display control unit 44 displays a difference between the image quality parameter before the change and the image quality parameter after the change on the display as information related to the change of the image quality parameter. The display control unit 44 may display a list of the image quality parameters on the display and may display the changed image quality parameter in an emphasized manner on the display. In addition, the display control unit 44 may display the image quality parameter before the change and the image quality parameter after the change on the display in a table format or a graph format. Information related to the change of the image quality parameter may be transmitted from the terminal apparatus 12 to the ultrasound diagnostic apparatus 10 and displayed on the display unit 22 of the ultrasound diagnostic apparatus 10.

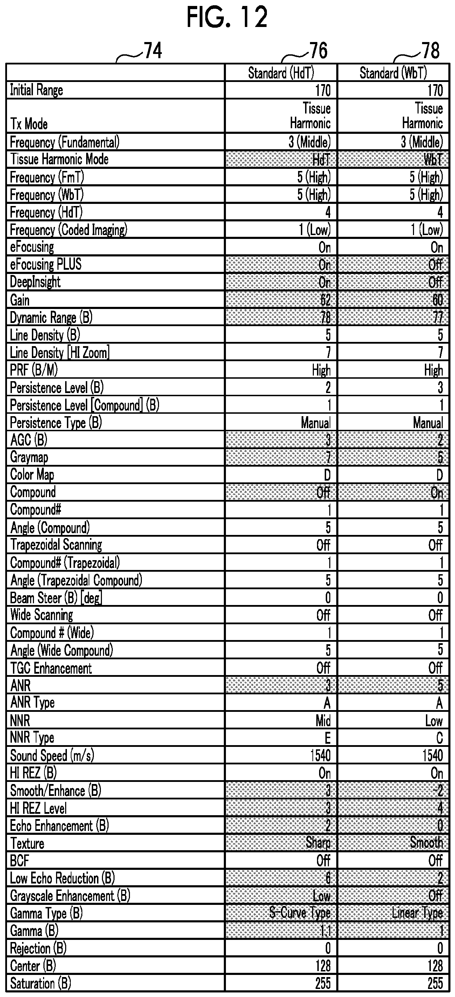

Fig. 12 shows a display example of information related to the change of the image quality parameter. In the example shown in Fig. 12, the display control unit 44 displays information related to the change of the image quality parameter on the display in a table format. The table is composed of a column 74 representing the name of the image quality parameter, and columns 76 and 78 representing the image quality parameter.

The display control unit 44 displays the changed image quality parameter and the unchanged image quality parameter in a distinguishable manner. For example, the display control unit 44 displays the changed image quality parameter in an emphasized manner by making a color of a display portion of the changed image quality parameter different from a color of a display portion of the unchanged image quality parameter. In the example shown in Fig. 12, hatching is performed on a portion displayed in an emphasized manner.

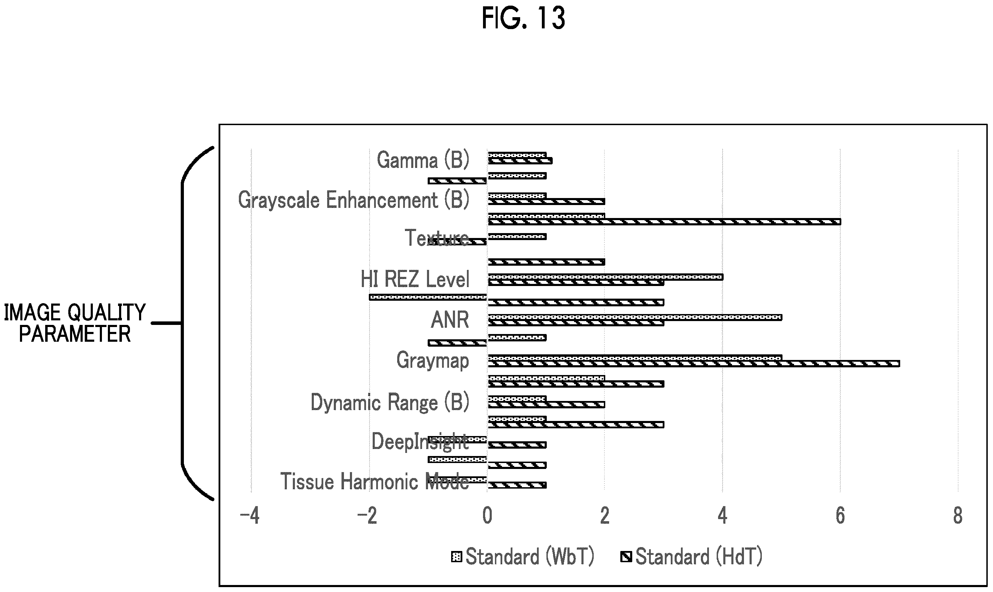

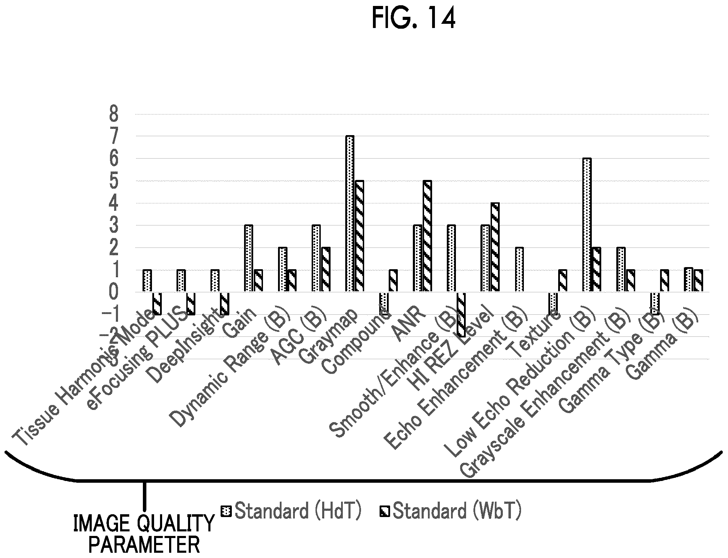

Figs. 13 and 14 show another display example. In the examples shown in Figs. 13 and 14, the display control unit 44 displays the information related to the change of the image quality parameter on the display in a graph. The value of each image quality parameter is represented by a bar graph. For example, the display control unit 44 creates the graphs shown in Figs. 13 and 14 by graphing the value of each image quality parameter shown in Fig. 12, and displays each graph on the display.

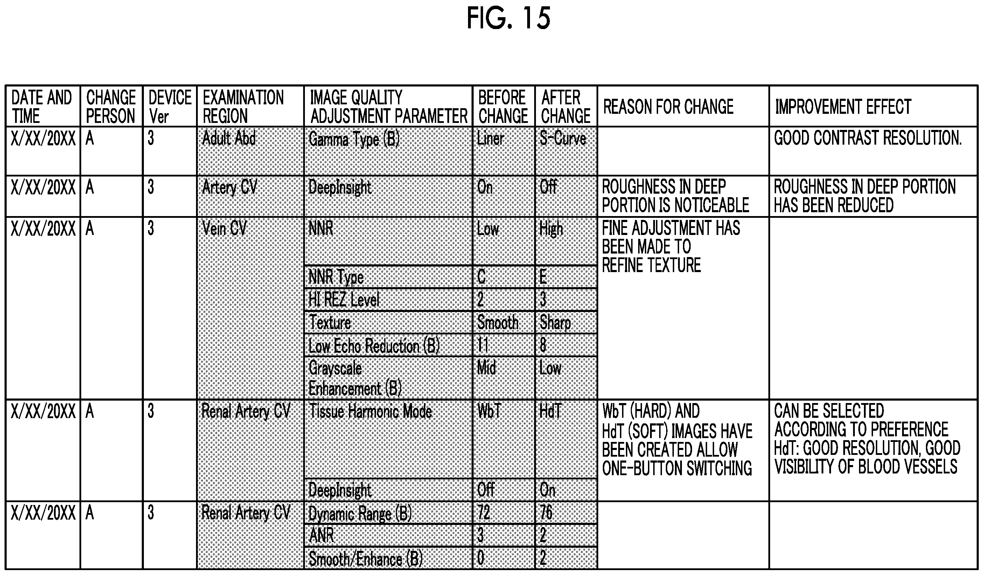

Fig. 15 shows still another display example. In the example shown in Fig. 15, the display control unit 44 lists the information related to the change of the image quality parameter and displays the information on the display. For example, the list includes a date and time at which the image quality parameter is changed, a name of a user who changes the image quality parameter, an examination region, a name of the image quality parameter, a value of the image quality parameter before the change, a value of the image quality parameter after the change, a reason for the change, and an improvement effect (effect of changing the image quality parameter). For example, the display control unit 44 displays the changed image quality parameter in an emphasized manner. In the example shown in Fig. 15, hatching is performed on a portion displayed in an emphasized manner.

The image generation unit 18, the display processing unit 20, and the control unit 30 can be realized by using hardware resources, such as a processor and an electronic circuit, and a device, such as a memory, may be used as necessary in the realization. In addition, the image generation unit 18, the display processing unit 20, and the control unit 30 may be realized by, for example, a computer. That is, the image generation unit 18, the display processing unit 20, and the control unit 30 may be realized by the cooperation between the hardware resources, such as a central processing unit (CPU) and a memory included in a computer, and the software (program) that defines the operation of the CPU or the like. The program is stored in the storage unit 26 of the ultrasound diagnostic apparatus 10 via a recording medium, such as a CD or a DVD, or via a communication path, such as a network. As another example, the image generation unit 18, the display processing unit 20, and the control unit 30 may be realized by a digital signal processor (DSP), an application specific integrated circuit (ASIC), a field programmable gate array (FPGA), or the like. Of course, a graphics processing unit (GPU) and the like may be used. The image generation unit 18, the display processing unit 20, and the control unit 30 may be realized by a single device or may be realized by a plurality of devices.

The reception unit 42, the display control unit 44, the storage control unit 46, the acquisition unit 48, and the recommendation unit 50 can be realized by using hardware resources such as a processor and an electronic circuit, and a device such as a memory may be used as necessary in the realization. In addition, the reception unit 42, the display control unit 44, the storage control unit 46, the acquisition unit 48, and the recommendation unit 50 may be realized by, for example, a computer. That is, all or a part of the reception unit 42, the display control unit 44, the storage control unit 46, the acquisition unit 48, and the recommendation unit 50 may be realized by cooperation between hardware resources, such as a central processing unit (CPU) or a memory included in a computer, and the software (program) that defines the operation of the CPU or the like. The program is stored in the storage unit 38 of the terminal apparatus 12 via a recording medium, such as a CD or a DVD, or via a communication path, such as a network. As another example, the reception unit 42, the display control unit 44, the storage control unit 46, the acquisition unit 48, and the recommendation unit 50 may be realized by a DSP, an ASIC, an FPGA, or the like. Of course, a GPU or the like may be used. The reception unit 42, the display control unit 44, the storage control unit 46, the acquisition unit 48, and the recommendation unit 50 may be realized by a single device or may be realized by a plurality of devices.

### Explanation of References

10: ultrasound diagnostic apparatus
12: terminal apparatus
42: reception unit
44: display control unit
46: storage control unit
48: acquisition unit
50: recommendation unit

## Claims

1. An ultrasound diagnostic system comprising:
a terminal apparatus (12); and
an ultrasound diagnostic apparatus (10),
wherein the terminal apparatus includes
a reception unit (42) that receives a preset condition, which is a predetermined image quality parameter set related to an image quality of an ultrasound image generated by the ultrasound diagnostic apparatus, from a user,
a terminal transmission unit (34) that transmits preset information indicating the preset condition received by the reception unit to the ultrasound diagnostic apparatus, and
a display control unit (44) that displays an image on a display of the terminal apparatus,
the ultrasound diagnostic apparatus includes
an image generation unit (18) that generates a plurality of pre-images from an ultrasound image in accordance with the preset condition indicated by the preset information transmitted from the terminal apparatus, and
an apparatus transmission unit (24) that transmits the plurality of pre-images to the terminal apparatus,
the display control unit displays the plurality of pre-images transmitted from the ultrasound diagnostic apparatus on the display as sample images, and
the terminal transmission unit transmits information related to a pre-image selected by the user from among the plurality of pre-images displayed on the display to the ultrasound diagnostic apparatus.

2. The ultrasound diagnostic system according to claim 1,
wherein the display control unit (44) displays a thumbnail image representing each of the plurality of pre-images transmitted from the ultrasound diagnostic apparatus on the display as the sample image, and
the terminal transmission unit (34) transmits the information related to a pre-image selected by a user by selecting the thumbnail image to the ultrasound diagnostic apparatus.

3. The ultrasound diagnostic system according to claim 1 or 2, further comprising:
a storage control unit (46),
wherein the storage control unit stores at least one of history information indicating a history of an image quality adjustment by the user, subject information related to a subject on which ultrasound waves are transmitted and received in order to generate the ultrasound image for generating the pre-image, or diagnosis condition information indicating a diagnosis condition in a case in which the ultrasound image for generating the pre-image is generated, and an image quality parameter set for generating the pre-image selected by the user in association with each other in a storage device.

4. The ultrasound diagnostic system according to claim 3,
wherein the terminal apparatus further includes an acquisition unit (48),
the acquisition unit acquires the image quality parameter set searched from the storage device by using at least one of the history information, the subject information, or the diagnosis condition information, and
the display control unit displays the ultrasound image of which the image quality is adjusted in accordance with the image quality parameter set acquired by the acquisition unit on the display.

5. The ultrasound diagnostic system according to claim 3 or 4,
wherein the terminal apparatus further includes a recommendation unit (50),
the recommendation unit specifies an image quality parameter set that is recommended to the user based on the image quality parameter set for generating the pre-image selected by the user, and the history of the image quality adjustment by the user, and
the display control unit displays a pre-image of which the image quality is adjusted in accordance with the image quality parameter set that is recommended to the user, on the display.

6. The ultrasound diagnostic system according to any preceding claim,
wherein the display control unit (44) displays information related to a change of the image quality parameter by the user on the display.
